# FASCICULE DE BREVET EUROPEEN

(11) **EP 2 968 197 B1**
(45) Date de publication et mention de la délivrance du brevet: **26.12.2018**
(21) Numéro de dépôt: 14713552.9
(22) Date de dépôt: 12.03.2014
(51) Int. Cl.: A61K 9/70

(54) **COMPOSITION FILMOGÈNE ET SON UTILISATION POUR LE TRAITEMENT DES AFFECTIONS CUTANÉES**
FILMBILDENDE ZUSAMMENSETZUNG UND VERWENDUNG DAVON ZUR BEHANDLUNG VON HAUTERKRANKUNGEN
FILM-FORMING COMPOSITION AND USE THEREOF FOR THE TREATMENT OF SKIN DISORDERS

(30) Priorité: 14.03.2013 FR 1352269
(43) Date de publication de la demande: 20.01.2016
(73) Titulaire: URGO RECHERCHE INNOVATION ET DEVELOPPEMENT, 21300 Chenôve (FR)
(72) Inventeur: DESFORGES, Sophie, F-21160 Perrigny les Dijon (FR)
(74) Mandataire: Cabinet Plasseraud
(86) Numéro de dépôt international: PCT/FR2014/050554
(87) Numéro de publication internationale: WO 2014/140475

(56) Documents cités:
- EP-A1- 0 770 373
- EP-A2- 0 412 705
- WO-A1-2010/086723
- WO-A2-2005/039499
- FR-A1- 2 929 119
- JP-A- H03 184 910
- US-A1- 2005 031 547

## Description

La présente invention concerne une composition liquide destinée à être appliquée sur la peau, les phanères ou les muqueuses, contenant au moins deux grades d'hydroxypropylcellulose dont l'un au moins présente un poids moléculaire d'au moins 800 000 et l'autre présente un poids moléculaire inférieur à 400 000, au moins un plastifiant, au moins un solvant organique et au moins un solvant inorganique. Une fois appliquée sur la peau, les phanères ou les muqueuses, les solvants s'évaporent et la composition forme un film solide protecteur sur la peau, les phanères ou les muqueuses pouvant être avantageusement utilisé dans le traitement des affections cutanées, en particulier de l'acné.

Les compositions filmogènes peuvent être formulées en phase aqueuse ou en phase solvant. Elles se présentent en général sous la forme d'un fluide que l'on applique sur la peau, les phanères ou les muqueuses par l'intermédiaire d'un applicateur approprié, tel un spray, un pinceau, un roll-on, une spatule, une palette ou un stylo. L'eau ou le solvant qu'ils contiennent s'évapore au contact de la peau, les phanères ou des muqueuses de manière à former un film solide qui protège la peau, les phanères ou les muqueuses dont les constituants pénètrent de façon très limitée dans la peau, les phanères ou les muqueuses. Ces compositions présentent l'avantage de protéger les tissus en évitant des contaminations bactériennes tout en les laissant respirer.

Les compositions filmogènes à base d'hydroxypropylcellulose contenant un solvant ont déjà été décrites dans l'art antérieur. Le document WO2005/039499 divulgue dans l'exemple 29: un film topique continu qui est formé par au moins deux grades de cellulose dérivée, comprenant Methocel E5 (HPMC MW 140.000) et Klucel JF (HPC MW140.000), sorbitol comme plastifiant, l'eau et éthanol. Au moins un autre grade de cellulose dérivée (Methocel E50 MW 30.000) est présent.

Le Document WO 2010086723 décrit une composition filmogène topique pour le traitement d'infections fongiques, comprenant comme polymère filmogène Klucel HF (HPC MW 850.000), éthanol et l'eau et optionnellement ajouter des plastifiants. Ainsi, le document US 5,081,158 décrit une composition filmogène à base d'hydroxypropylcellulose (HPC), de solvant volatil et d'acide carboxylique faible. Cette composition est destinée, en particulier, aux muqueuses et porte plus particulièrement sur une méthode de traitement des aphtes.

Cependant, les formules décrites dans ce brevet présentent plusieurs inconvénients. Leur tenue dans le temps, en particulier lorsqu'elles sont appliquées sur la peau n'est pas satisfaisante. Le film formé n'est pas esthétique : il présente très rapidement des craquelures.

Aussi, le film formé n'est pas souple, il ne suit pas les aspérités et les mouvements de la peau et provoque ainsi une gêne.

La Demanderesse a donc souhaité développer une composition filmogène qui puisse recouvrir de façon satisfaisante une zone de peau, tout en formant un film continu, souple, conformable et d'épaisseur suffisante. Le produit de l'invention peut être avantageusement appliqué sur une zone de la peau soumise à des mouvements, des tensions ou des élongations, notamment au niveau du visage ou des articulations.

La Demanderesse a découvert qu'en associant au moins deux grades d'hydroxypropylcellulose dont l'un au moins présente un poids moléculaire d'au moins 800 000 et l'autre présente un poids moléculaire inférieur à 400 000, au moins un plastifiant, au moins un solvant organique et au moins un solvant inorganique dans des proportions particulières, on obtient une composition formant un film homogène sur la peau qui présente une bonne tenue dans le temps, dont l'aspect esthétique est amélioré. Aussi, en associant le solvant inorganique à un solvant organique, on obtient une composition dont le temps de séchage pour former un film est rapide et qui, lorsqu'elle est appliquée sur une peau lésée, provoque une douleur restant acceptable.

La Demanderesse a ainsi élaboré une nouvelle base de composition filmogène dotée d'une bonne tenue dans le temps, d'une résistance aux frottements satisfaisante. Les films sont en outre souples et procurent un confort suffisant à l'utilisateur notamment lorsque la composition est appliquée sur une zone soumise aux mouvements, tensions ou élongations.

La présente invention a donc pour objet une composition topique filmogène contenant au moins deux grades d'hydroxypropylcellulose dont l'un au moins présente un poids moléculaire d'au moins 800 000 et l'autre présente un poids moléculaire inférieur à 400 000, au moins un plastifiant, au moins un solvant organique et au moins un solvant inorganique, caractérisée en ce que :
(a) l'hydroxypropylcellulose présentant un poids moléculaire d'au moins 800 000 représente de 0,2 à 4 % en poids du poids total de la composition,
(b) l'hydroxypropylcellulose présentant un poids moléculaire inférieur à 400 000 représente 0,2 à 20% en poids du poids total de la composition,
(c) le plastifiant représente de 0,5 à 10% en poids du poids total de la composition,
(d) le solvant inorganique représente 10 à 60% en poids du poids total de la composition,
(e) le solvant organique représente 40 à 80% en poids du poids total de la composition.

Par « composition topique » on entend une composition destinée à être appliquée sur la peau, les phanères ou les muqueuses.

De préférence, l'hydroxypropylcellulose présentant un poids moléculaire supérieur à 800 000 est présente en une teneur allant 1 à 3% en poids du poids total de la composition.

De préférence encore, l'hydroxypropylcellulose présentant un poids moléculaire inférieur à 400 000 est présente en une teneur allant de 2 à 6% en poids du poids total de la composition.

### Polymère filmogène

Outre les deux grades d'hydroxypropylcellulose la composition selon l'invention peut comprendre d'autres polymères filmogènes. Selon la présente invention, on entend par "polymère filmogène", un polymère apte à former à lui seul ou en présence d'un agent auxiliaire de filmification, un film continu et adhérent sur un support, notamment sur la peau, les muqueuses ou les phanères.

Parmi ces polymères, on peut citer, à titre d'exemple, les polymères synthétiques, de type radicalaire ou de type polycondensat, les polymères d'origine naturelle et leurs mélanges.

Les polymères d'origine naturelle peuvent être, par exemple, les gommes de guar, les gommes arabiques, les alginates, la gomme de xanthane, la gélatine, le chitosan, les silicates, les silices hydratées, les polymères cellulosiques autres que l'hydroxypropylcellulose ainsi que les dérivés de ces polymères.

Les polymères cellulosiques peuvent être choisis parmi l'hydroxypropylméthylcellulose, les éthers de cellulose, les esters de celluloses, les nitrocelluloses, l'acétate de cellulose, l'acétobutyrate de cellulose, l'acétopropionate de cellulose, l'éthyl cellulose, les dérivés cellulosiques solubles ou insolubles dans l'eau, et leurs mélanges.

Les polymères filmogènes peuvent également être choisis parmi les polyuréthanes, les polyesters, les polyesters amides, les polyamides, les polyurées, les polymères vinyliques, les polymères acryliques, les polyvinylbutyrals, les résines alkydes, les résines issues des produits de condensation d'aldéhyde telles que les résines arylsulfonamide formaldéhyde, les résines aryl-sulfonamide époxy, les résines éthyltosylamide, les résines acétophénone/formaldéhyde,le copolymère de PolyVinylMéthyl éther et d'Anhydride Maléique (PVM/MA) et leurs mélanges.

Les polymères vinyliques peuvent résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styréniques comme le styrène et l'alpha-méthyl styrène. Les polymères acryliques peuvent être des homopolymères de l'acide acrylique, des copolymères de l'acide acrylique, des homopolymères de l'acide méthacrylique ou des copolymères de l'acide méthacrylique.

### Le plastifiant

La composition filmogène selon l'invention comprend au moins un plastifiant. En particulier, on peut citer, seuls ou en mélange, les plastifiants usuels, tels que :
- les alcools gras comme l'octyldodécanol, le 2-butyloctanol, le 2-hexyl décanol, le 2-undécylpentadécanol, l'alcool oléique ;
- les glycols et leurs dérivés, tels que la glycérine, le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les acides gras tels que l'acide oléique, l'acide linoléique, l'acide linolénique ;
- les esters de glycol tels que la triacétine (ou triacétate de glycéryle) ;
- les dérivés de propylène glycol et en particulier le propylène glycol phényléther, le propylène glycol diacétate, le dipropylène glycol éthyléther, le tripropylène glycol méthyléther, le propylène glycol butyléther ;
- les esters d'acides, notamment carboxyliques, tels que les citrates, les phtalates, les adipates, les carbonates, les tartrates, les phosphates, les sébacates et en particulier les esters d'acide monocarboxylique tels que l'isononanoate d'isononyle, l'érucate d'oléyle ou le néopentanoate d'octyl-2-docécyle ;
- les dérivés oxyéthylénés, tels que les huiles oxyéthylénées, notamment les huiles végétales, telles que l'huile de sésame, l'huile de ricin, l'huile d'amande, l'huile de canola, l'huile de noisette, l'huile de pistache, l'huile de lin, l'huile de bourrache, l'huile de chanvre, l'huile de jojoba, l'huile de tournesol, l'huile de germe de blé, l'huile de maïs et/ou de germe de maïs, l'huile d'arachide, l'huile d'avocat, l'huile de carthame, l'huile de colza, l'huile d'olive, l'huile d'argan, l'huile de tournesol, l'huile de pépin de raisin, l'huile de soja, l'huile de noix, l'huile de pépin de courge, l'huile de palme, l'huile de coprah, et leurs mélanges. L'huile peut également être un dérivé d'une des huiles végétales citées précédemment. Il peut s'agir d'huile hydrogénée ou non, peroxydée ou non ;
et leurs mélanges.

Selon un mode préféré de réalisation, le plastifiant est choisi parmi les glycols, notamment la glycérine. En effet la glycérine présente l'avantage d'assouplir le film, d'hydrater la peau (ou la muqueuse) et de favoriser la diffusion du (des) actif(s) lorsque la composition en contient.

La teneur en plastifiant peut aller de 0,5 % à 10% en poids, et en particulier de 2 à 5 % en poids, par rapport au poids total de la composition.

### Les solvants

Les compositions de l'invention contiennent au moins un solvant organique et au moins un solvant inorganique.

Ces solvants permettent de solubiliser une partie ou la totalité des ingrédients de la composition et participe à la formation d'un film sur la peau lors de son application.

Comme solvant organique utilisable dans le cadre de la présente invention, on peut citer :
- les cétones telles que la méthyléthylcétone, la méthylisobutylcétone, la diisobutylcétone, l'isophorone, la cyclohexanone, l'acétone;
- les alcools tels que l'éthanol, l'isopropanol, le n-propanol, le n-butanol, le diacétone alcool, le 2-butoxyéthanol, le cyclohexanol;
- les éthers de propylène glycol tels que le monométhyléther de propylène glycol, l'acétate de monométhyl éther de propylène glycol, le mono n-butyl éther de dipropylène glycol;
- les esters tels que l'acétate d'éthyle, l'acétate de méthyle, l'acétate de propyle, l'acétate de n-butyle, l'acétate d'isopentyle;
- les éthers tels que le diéthyléther, le diméthyléther ou le dichlorodiéthyléther; et
- leurs mélanges.

Le solvant organique représente 40 à 80% en poids du poids total de la composition, préférentiellement de 50 à 70% en poids du poids total de la composition.

Selon un mode préféré de réalisation, le solvant organique est volatil.

Par " solvant organique volatil", on entend un solvant organique susceptible de s'évaporer au contact de la peau en moins d'une heure, à température ambiante et pression atmosphérique. Le solvant organique volatil est liquide à température ambiante, présente notamment une pression de vapeur non nulle à température ambiante et pression atmosphérique, en particulier il présente une pression de vapeur allant de 0,13 Pa à 40 000 Pa (10⁻³ à 300 mm de Hg), et de préférence allant de 1,3 Pa à 8000 Pa (0,01 à 60 mm de Hg).

Selon un mode particulièrement préféré de réalisation, le solvant organique est choisi parmi les alcools liquides à température ambiante tels que l'éthanol, l'isopropanol, le diacétone alcool, le 2-butoxyéthanol, le cyclohexanol et leurs mélanges, et de préférence l'éthanol.

Le solvant inorganique représente quant à lui 10 à 60% en poids du poids total de la composition, préférentiellement de 20 à 40% en poids du poids total de la composition.

Le solvant inorganique sera avantageusement l'eau.

La Demanderesse a constaté que de manière avantageuse, lorsqu'on associait ces deux solvants dans de telles proportions, on obtenait un bon compromis entre le temps de séchage et la douleur ressentie lors de l'application de la composition sur peau lésée. En effet, l'éthanol présente l'avantage de se volatiliser rapidement mais provoque une douleur à l'application. En ajoutant de l'eau dans cette proportion, on diminue la douleur tout en ayant un temps de séchage acceptable (notamment inférieur à 120 secondes).

Les meilleurs résultats sont obtenus lorsque le rapport pondéral solvant organique/solvant inorganique est compris entre 0,7 et 8, de préférence entre 1,5 et 4.

Un autre avantage d'utiliser un mélange de solvant organique et solvant inorganique est que l'on peut dissoudre un plus grand nombre de composés, tels que par exemple des actifs.

### Additifs

La composition selon l'invention peut comprendre un ou plusieurs additifs pharmaceutiquement acceptables, comme par exemple les parfums, les arômes, les colorants, les pigments, les agents matifiants, les agents rhéologiques, les conservateurs, les vitamines, les huiles essentielles et les agents actifs, notamment choisis parmi les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques et les filtres solaires.

En particulier, les actifs pouvant être introduits dans la composition selon l'invention peuvent être choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que l'Aciclovir, le Famciclovir, le Ritonavir ;
- les antifongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine ;
- les anti-douleurstels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, les extraits de CentellaAsiatica, la papaïne, les silicones, les huiles essentielles de thym, de niaouli, de romarin, d'arbre à thé et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, l'Allantoïne ;
- les agents hydratants tels que l'acide hyaluronique, l'urée, le glycérol, les acides gras, modulateurs des aquaporines, les huiles végétales, le chitosan, certains sucres dont le sorbitol, les beurres et les cires ;
- les agents dépigmentants tels que l'acide kojique (KojicAcid SL® - Quimasso (Sino Lion)), l'Arbutine (Olevatin® - Quimasso (Sino Lion)), le mélange de palmitoylpropyl de sodium et d'extrait de nénuphar blanc (Sepicalm® - Seppic), l'undécylénoyl phénylalanine (Sepiwhite® - Seppic), l'extrait de réglisse obtenue par fermentation d'Aspergillus et éthoxydiglycol (GatulineWhitening® - Gattefossé), l'acide octadécènedioïque (ODA White® - Sederma), l'alpha-arbutin (Alpha-arbutin®, SACI-CFPA (Pentapharm)), l'extrait aqueux de feuilles Arctophylos Uva Ursi (Melfade-J® - SACI-CFPA (Pentapharm)), le mélange de plante complexe Gigawhite® (SACI-CFPA (Alpaflor)), la diacétylboldine (Lumiskin® - Sederma), l'extrait de mandarine du Japon (Melaslow® - Sederma), le mélange d'extrait de citron enrichi en acide citrique et d'extrait de concombre (Uninontan®U-34 - Unipex), le mélange d'extrait de Rumex occidentalis et de vitamine C (Tyrostat® 11
   - Unipex), des oligopeptides (Mélanostatine 5® - Unipex), le dipalmitatekojique (KAD-15® - Quimasso (Sino Lion)), le complexe d'origine naturelle Vegewhite® de LCW, des extraits de germe de blé (Clariskin® II - Silab), l'éthyldiaminetriacétate (EDTA);
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline® (Sederma), les protéases obtenues par fermentation de Bacillus Subtilis, le produit Linked-Papain® (SACI-CFPA), la papaïne (enzyme protéolytique issue du fruit de papaye);
- les actifs restructurants (par exemple resctructurants des phanères) tels que les dérivés de silice, la vitamine E, la camomille, le calcium, l'extrait de prêle, le Lipester de soie;
- les anesthésiques tels que la benzocaïne, la lidocaïne, la dibucaïne, le chlorhydrate de pramoxine, la bupivacaïne, la mepivacaïne, la prilocaïne, l'étidocaïne ;
- les filtres solaires, tels que les filtres chimiques (Oxybenzone, Sulisobenzone, Dioxybenzone, Tinosorb S®, Avobenzone, p-méthoxycinnamate de 2-éthoxyéthyle, Uvinul® A+, Mexoryl® XL, Méthoxycinnamate ou octinoxate d'octyle, Salicylate ou octisalate d'octyle, octyl triazone ou Uvinul® T 150, salicylate de méthyle, meradimate, enzacamène, MBBT ou Tinosorb® M, cyanophénylcinnamate d'octyleouParsol® 340, Acide para-aminobenzoïque, Ensulizole, Parsol® SLX ou Polysiloxane-15 ou Benzylidènemalonatepolysiloxane, salicylate de triéthanolamine ou salicylate de trolamine, Mexoryl® SX ou acide téréphtalylidènedicamphosulfonique) et les filtres minéraux (oxydes de Zinc, dioxyde de titane, kaolin, ichtyol).

De manière préférentielle, lorsque la composition est utilisée dans le traitement de l'acné, elle comprend un agent kératolytique, de préférence l'acide salicylique en association avec un actif favorisant la cicatrisation, de préférence l'huile essentielle d'arbre à thé.

Comme indiqué précédemment, la composition selon la présente invention se présente sous forme d'un liquide destiné à être appliqué à l'aide d'un applicateur approprié, tel qu'un pinceau, une palette, un spray, un roll-on, une spatule ou un stylo.

### Utilisation de la composition

Les compositions selon la présente invention sont destinées à être appliquées sur la peau, les phanères ou les muqueuses. Elles peuvent être avantageusement utilisées sur des plaies ou cicatrices, qu'elles soient liées à un accident, une maladie ou les suites d'une intervention chirurgicale, des brûlures, ampoules, gerçures ou crevasses, dans le traitement des affections cutanées telles que, à titre d'exemple, l'acné, la varicelle, le zona, la couperose, les brûlures au premier degré, l'eczéma, l'hyperpigmentation, la lucite, le vitiligo, la xérose, la porphyrie, les vergetures, le psoriasis, les piqûres d'insectes, l'herpès, les aphtes.

L'invention a donc pour objet la composition telle que définie ci-dessus pour son utilisation dans la protection ou le traitement des plaies, des cicatrices, des brûlures, des ampoules, des mycoses, des gerçures, des crevasses, l'acné, la varicelle, le zona, la couperose, l'eczéma, l'hyperpigmentation, la lucite, le vitiligo, la xérose, la prophyrie, les vergetures, le psoriasis, les piqûres d'insectes, l'herpès, les aphtes.

L'invention a notamment pour objet la composition telle que définie ci-dessus pour son utilisation dans une méthode de traitement de l'acné.

L'acné est une dermatose inflammatoire des follicules pilosébacés (glandes sécrétant le sébum, à la racine des poils) avec formation de comédons.

L'acné est une maladie de la peau et plus précisément des follicules pilo-sébacées. Lors de cette affection, le canal pilaire se retrouve obstrué par du sébum et des cellules mortes, entrainant diverses lésions consécutives à la rétention du sébum, à l'inflammation ou à l'infection des follicules pilo-sébacés.

*Propionibacterium acnes* est une bactérie qui vit normalement, chez tout le monde, dans les follicules. Elle ne provoque pas d'infection, mais aggrave l'inflammation du follicule lorsqu'il y a un excès de sébum, à l'origine des boutons rouges.

Parmi les traitements connus, on peut citer les crèmes ou pommades. Elles sont essentiellement à base de peroxyde de benzoyle et sont disponibles en parapharmacie (sans prescription médicale). Ils sont au moins aussi efficaces que les antibiotiques et semblent plus actifs que les rétinoïdes locaux dans les formes inflammatoires. Le peroxyde de benzoyle peut être irritant à des concentrations élevées, ces dernières n'étant pas plus efficaces qu'à des concentrations moindres. Il existe des produits qui s'attaquent à la bactérie (*Propionibacterium acnes*)*,* d'autres agissent sur les médiateurs de l'inflammation, comme la nicotinamide, une molécule présente naturellement dans de nombreux aliments. D'autre part, le zinc permet de réduire la sécrétion de sébum. Les rétinoïdes locaux sont efficaces dans les formes inflammatoires et sur les comédons. Ils sont cependant irritants en début de traitement, avec parfois un eczéma (rarement), une photosensibilité. Ils sont tératogènes (malformation possible d'un foetus avant la naissance) et nécessitent la prise d'un contraceptif chez la femme.

En appliquant la composition selon l'invention sur une éruption cutanée dès les premiers signes annonciateurs de la lésion, il est possible de limiter la formation des comédons, papules, pustules ou nodules. Lorsqu'elle est appliquée après la formation des comédons, la composition selon l'invention contribue à limiter la formation des papules, pustules ou nodules.

Enfin, le film obtenu avec les compositions de l'invention permet également de favoriser la cicatrisation des comédons, papules, pustules et nodules.

### Méthode de traitement cosmétique

La présente invention porte également sur une méthode de traitement cosmétique de la peau, comprenant au moins une application de la composition selon la présente invention sur des imperfections cutanées toutes les 24 heures en vue d'améliorer l'apparence de la peau.

La présente invention est illustrée plus en détails dans les exemples non limitatifs décrits ci-après.

### Exemples 1 à 19 de compostions selon l'invention et comparatives

**Les formulations des exemples 1 à 19 ont été réalisées en suivant le protocole suivant :**
Les actifs (exemple 19) sont ajoutés dans l'éthanol sous agitation maintenue pendant 15 min. Puis, on ajoute l'eau et la glycérine (toujours sous agitation).

L'hydroxypropylcellulose de poids moléculaire inférieur à 400 000 (Klucel GF, JF ou LF) est ajoutée en pluie au mélange puis est laissée sous agitation pendant environ une heure (exemples 2 à 5, 7 à 17).

Une fois que ce grade d'hydroxypropylcellulose est bien incorporé, on ajoute l'hydroxypropylcellulose de haut poids moléculaire (Klucel MF) en pluie puis on laisse sous agitation pendant environ deux heures.

L'agitation est ensuite stoppée puis on laisse au repos environ 12 heures afin de permettre à l'hydroxypropylcellulose de finir de gonfler avant le conditionnement du produit final.

| | | | **Exemples 1 à 7 comparatifs** | | | | | | |
|---|---|---|---|---|---|---|---|---|---|
| | | | **Ex. Comp.1** | **Ex. Comp.2** | **Ex. Comp.3** | **Ex. Comp.4** | **Ex. Comp.5** | **Ex. Comp.6** | **Ex. Comp.7** |
| **Nom commercial** | **Fabricant/Fournisseur** | **Nom INCI** | **% massique** | **% massique** | **% massique** | **% massique** | **% massique** | **% massique** | **% massique** |
| Klucel MF | HERCULES | HPC (*) | 5 | 2 | 2 | 2 | 2 | 6 | |
| Klucel GF | HERCULES | HPC (*) | | | 3 | | | | |
| Klucel JF | HERCULES | HPC (*) | | | | 3 | | | |
| Klucel LF | HERCULES | HPC (*) | | | | | 3 | | 6 |
| Glycerine 4810 | Sigma-Aldrich | glycérol | 0 | 0 | 0 | 0 | 0 | **0** | **0** |
| Ethanol absolu | Charbonneau Brabant | Alcohol | 70 | 72 | 70 | 70 | 70 | 69 | 69 |
| Eau | | | 25 | 26 | 25 | 25 | 25 | 25 | 25 |

| | | | | | | | | | |
|---|---|---|---|---|---|---|---|---|---|
| * HPC = Hydroxypropyl-cellulose | | | | | | | | | |

### Test de tenue dans le temps des compositions filmogènes

Un panel de 10 personnes a testé les compositions des exemples comparatifs 1 à 7 sur les critères suivants :
- Formation d'un film et sensation de protection après séchage
- Tenue du film à 1h, 2h, 4h et 8h

Les panelistes sont convoqués le matin, ils appliquent sur leurs joues avec une spatule silicone les formules 1 à 7 (1, 3, 5 et 6 sur la joue droite et 2 et 4 et 7 sur la joue gauche).

| **Temps d'évaluation** | **Question** | **Exemples 1 et 6 comparatifs** | **Exemple 2 comparatif** | **Exemples 3 à 5 comparatifs** | **Exemple 7 comparatif** |
|---|---|---|---|---|---|
| **Juste après séchage** | **Formation et aspect du film** | Formules difficile à déposer, trop visqueuses ne sèchent pas | 90% des personnes ne perçoivent pas de film après séchage | 100 % des personnes perçoivent un film après séchage Film discret | 100 % des personnes trouvent la formule difficile à poser du fait de sa trop faible viscosité. Pas assez de produit déposé pour percevoir un film après application |
| **1h après** | **Présence du film** | - | 100 % Non | 50 à 70 % Oui 30 à 50 % du panel a retiré le film car il y avait un décollement trop important. | - |
| | **Aspect du film** | - | Absence | Le film est encore présent mais décollement sur les bords et présence de craquelures | - |
| **2 h après** | **Présence du film** | - | - | 80 à 90 % Non 10 à 20 % Oui | - |
| | **Aspect du film** | - | - | Les films sont soit tombés tout seuls soit ont été retirés car trop décollés ou trop craquelés avec aspect inesthétique. Le film restant est vraiment très craquelé | - |
| **4 h après** | **Présence du film** | - | - | Tous les films sont tombés ou ont été retirés | - |
| | **Aspect du film** | - | - | - | - |
| **8 h après** | **Présence du film** | - | - | - | - |
| | **Aspect du film** | - | - | - | - |

Les formules comparatives obtenues avec un seul polymère sont ainsi soit trop visqueuses pour permettre une application acceptable (exemple 1 et 6), soit ne laissent pas un film suffisamment épais pour être perçu (exemple 2 et 7).

Le mélange de deux Klucel de grades différents (exemples 3 à 5) permet de former un film physique perceptible par le panéliste mais qui ne tient pas suffisamment longtemps car il craquèle et se décolle sur les bords entrainant un retrait spontané des panélistes.

On a préparé de nouvelles compositions selon l'invention et comparatives, selon la méthode décrite précédemment :

| | | | **Exemples 8 à 13** | | | | | |
|---|---|---|---|---|---|---|---|---|
| | | | **Exemple 8 comparatif** | **Exemple 9 Inv.** | **Exemple 10 Inv.** | **Exemple 11 Inv.** | **Exemple 12 comparatif** | **Exemple 13 comparatif** |
| **Nom commercial** | **Fabricant Fournisseur** | **Nom INCI** | **% massique** | **% massique** | **% massique** | **% massique** | **% massique** | **% massique** |
| Klucel MF | HERCULES | HPC (*) | 2 | 1 | 1 | 1 | 1 | 1 |
| Klucel GF | HERCULES | HPC (*) | | 5 | | | | |
| Klucel JF | HERCULES | HPC (*) | | | 5 | | | |
| Klucel LF | HERCULES | HPC (*) | | | | 5 | 5 | 5 |
| Glycerine 4810 | Sigma-Aldrich | glycérol | 3 | 3 | 3 | 3 | **15** | **0,3** |
| Ethanol absolu | Charbonneau Brabant | Alcohol | 70 | 67 | 67 | 67 | 58 | 69 |
| Eau | | | 25 | 24 | 24 | 24 | 21 | 24,7 |

| | | | | | | | | |
|---|---|---|---|---|---|---|---|---|
| * HPC = Hydroxypropyl-cellulose | | | | | | | | |

### Test de tenue dans le temps des compositions filmogènes

Un panel de 10 personnes a testé les compositions des exemples 8 à 13 sur les critères suivants:
- Formation d'un film et sensation de protection après séchage
- Tenue du film à 1h, 2h, 4h et 8h
- Aspect du film formé

Les panelistes sont convoqués le matin, ils appliquent sur leurs joues avec une spatule silicone les formules 8 à 13 (8, 10 et 12 sur la joue droite et 9, 11 et 13 sur la joue gauche).

| **Temps d'évaluation** | **Question** | **Exemple 8 comparatif** | **Exemples 9 à 11 Invention** | **Exemple 12 comparatif** | **Exemple 13 comparatif** |
|---|---|---|---|---|---|
| **Juste après séchage** | **Formation et aspect du film** | Pas de sensation de présence d'un film | Formation d'un film souple et très discret | Le film ne sèche pas et reste collant. Retrait immédiat | Formation d'un film discret après séchage |
| **1h après** | **Présence du film** | - | 100 % film encore présent | - | 90 % film présent 10 % film retiré car trop décollé |
| | **Aspect du film** | - | Film discret, souple et confortable | - | Films craquelés et décollés sur les bords |
| **2 h après** | **Présence du film** | - | 100 % film encore présent | - | 90 % film retiré car inesthétique ou trop décollé |
| | **Aspect du film** | - | Film discret, souple et confortable | - | Film craquelé, blanc, décollé sur une grande surface |
| **4 h après** | **Présence du film** | - | 100 à 90 % film encore présent | - | 100 % films absent |
| | **Aspect du film** | - | Film discret, souple et confortable | - | - |
| **8 h après** | **Présence du film** | - | 80 à 70 % film encore présent | - | - |
| | **Aspect du film** | - | Film discret, souple et confortable. | - | - |

Les formules contenant de la glycérine permettent une meilleure tenue dans le temps des films formés (exemples 9 à 11).

La glycérine avec un seul grade d'hydroxypropylcellulose ne permet pas la formation d'un film protecteur (exemple 8).

Une concentration élevée en glycérine empêche le film de sécher, il reste collant (exemple 12).

Une concentration trop faible de glycérine ne permet pas de rendre le film souple, celui-ci se craquèle et tombe rapidement (exemple 13).

On a préparé de nouvelles compositions selon l'invention et comparatives, selon la méthode décrite précédemment :

| | | | **Exemples 14 à 18** | | | | |
|---|---|---|---|---|---|---|---|
| | | | **Exemple 14 comparatif** | **Exemple 15 Inv.** | **Exemple 16 Inv.** | **Exemple 17 comparatif** | **Exemple 18 Inv.** |
| **Nom commercial** | **Fabricant Fournisseur** | **Dénomination INCI** | **% massique** | **% massique** | **% massique** | **% massique** | **% massique** |
| Klucel MF | HERCULES | HPC (*) | 1 | 1 | 1 | 1 | 1 |
| Klucel GF | HERCULES | HPC (*) | | | | | |
| Klucel JF | HERCULES | HPC (*) | | | | | |
| Klucel LF | HERCULES | HPC (*) | 5 | 5 | 5 | 5 | 5 |
| Glycerine 4810 | Sigma-Aldrich | glycérol | 3 | 3 | 3 | 3 | 3 |
| Ethanol absolu | Charbonneau Brabant | Alcohol | **24** | 40 | 80 | **90** | 60 |
| Eau | | | **67** | 51 | 11 | **1** | 31 |

| | | | | | | | |
|---|---|---|---|---|---|---|---|
| * HPC = Hydroxypropyl-cellulose | | | | | | | |

### Test de temps de séchage des compositions filmogènes et de douleur à l'application

Un panel de 6 personnes présentant la peau des mains irritée pas le froid a testé les compositions des exemples 14 à 18 sur les critères suivants:
- Temps de séchage du film
- Douleur à l'application

Les panelistes sont convoqués le matin, ils appliquent sur leurs mains avec une spatule silicone les formules 14 à 18 (14, 16 et 18 sur la main droite et 15 et 17 sur la main gauche).

Les résultats sont présentés dans le tableau suivant :

| **Evaluation** | | **Exemple 14 comparatif** | **Exemple 15 Inv.** | **Exemple 16 Inv.** | **Exemple 17 Comparatif** | **Exemple 18 Inv.** |
|---|---|---|---|---|---|---|
| Temps de séchage | >5 min | 6/6 | 1/6 | | | |
| | 2 min - 5 min | | 4/6 | 1/6 | | 1/6 |
| | < 2 min | | | 5/6 | 6/6 | 5/6 |
| Douleur à l'application | Douleur inacceptable | | | 2/6 | 4/6 | |
| | Douleur vive mais acceptable | | 4/6 | 3/6 | 2/6 | 4/6 |
| | Douleur acceptable | 6/6 | 2/6 | 1/6 | | 2/6 |
| | Aucune douleur | | | | | |

Une teneur en eau trop élevée associée à une teneur en éthanol trop faible empêche le séchage du film. Un temps de séchage > à 5 min n'est pas acceptable (exemple 14).

Une teneur en eau trop faible associée à une teneur en éthanol trop élevée joue en faveur du temps de séchage. Cependant son augmentation peut être défavorable vis-à-vis de la douleur ressentie (exemple 17).

Il faudra donc trouver un compromis acceptable afin de lier les deux attentes. Les exemples 15, 16 et 18 proposent ainsi un meilleur compromis, l'exemple 18 donnant les meilleurs résultats parmi toutes les formules testées.

| **Exemple 19** | | | |
|---|---|---|---|
| **Nom commercial** | **Fabricant/ Fournisseur** | **Dénomination INCI** | **% massique** |
| Klucel MF | HERCULES | hydroxypropylcellulose | 1,5 |
| Klucel LF | HERCULES | hydroxypropylcellulose | 4 |
| Glycerine 4810 | Sigma - Aldrich | glycérol | 3 |
| Ethanol absolu | Charbonneau Brabant | Alcohol | 60,5 |
| Eau | | | 30,7 |
| Acide salicylique | Rhodia | Salicylicacid | 0,15 |
| Huile essentielle d'arbre à thé | | TeatreeoilAustralia | 0,15 |

Le mélange de deux actifs : acide salicylique à 0,15 % et huile essentielle d'arbre à thé à 0,15 % a montré une efficacité intéressante sur la souche *Propionobacter acnes* selon la norme NF EN 1040 Avril 2006 avec comme neutralisant :

| | |
|---|---|
| Polysorbate 80 | 30,0 g |
| Saponine | 30,0 g |
| L- Histidine | 1,0 g |
| Lecithine | 3,0 g |
| Thiosulfate de sodium | 5,0 g |
| Chlorure de sodium | 8,5 g |
| Tryptone | 1,0 g |
| Eau distillée | 1000,0 ml |

Stérilisé à l'autoclave à une température de 121-0/+3 °C pendant au moins 15 minutes.

L'inoculum utilisé est de 2,2.108 UFC/mL d'une souche de Propionibacterium acnes CIP 53.117T.

L'incorporation de ce mélange dans les formules testées précédemment pour leurs propriétés de formation de film, tenue dans le temps, séchage rapide et application peu douloureuses sur peau abîmée présente un intérêt particulier dans le traitement des boutons d'acné.

## Revendications

1. Composition topique filmogène comprenant au moins deux grades d'hydroxypropylcellulose dont l'un au moins présente un poids moléculaire d'au moins 800 000 et l'autre présente un poids moléculaire inférieur à 400 000, au moins un plastifiant, au moins un solvant organique et au moins un solvant inorganique, **caractérisée en ce que** :
(a) l'hydroxypropylcellulose présentant un poids moléculaire d'au moins 800 000 représente de 0,2 à 4 % en poids du poids total de la composition,
(b) l'hydroxypropylcellulose présentant un poids moléculaire inférieur à 400 000 représente 0,2 à 20% en poids du poids total de la composition,
(c) le plastifiant représente de 0,5 à 10% en poids du poids total de la composition,
(d) le solvant inorganique représente 10 à 60% en poids du poids total de la composition,
(e) le solvant organique représente 40 à 80% en poids du poids total de la composition.

2. Composition selon la revendication 1, **caractérisée en ce que** l'hydroxypropylcellulose présentant un poids moléculaire d'au moins 800 000 est présente en une teneur allant de 1 à 3% en poids, et l'hydroxypropylcellulose présentant un poids moléculaire inférieur à 400 000 en poids est présente en une teneur allant de 2 à 6% en poids par rapport au poids total de la composition.

3. Composition selon l'une des revendications 1 ou 2, **caractérisée en ce que** le plastifiant est choisi parmi les glycols, notamment la glycérine.

4. Composition selon l'une quelconque des revendications précédentes, **caractérisée en ce que** le solvant organique représente de 50 à 70% en poids du poids total de la composition.

5. Composition selon l'une quelconque des revendications précédentes, **caractérisée en ce que** le solvant inorganique représente de 20 à 40% en poids du poids total de la composition.

6. Composition selon l'une quelconque des revendications précédentes, **caractérisée en ce que** le solvant organique est choisi parmi les alcools liquides à température ambiante tels que l'éthanol, l'isopropanol, le diacétone alcool, le 2-butoxyéthanol, le cyclohexanol et leurs mélanges, et de préférence l'éthanol.

7. Composition selon l'une quelconque des revendications précédentes, **caractérisée en ce qu'**elle comprend des additifs pharmaceutiquement acceptables, tels que les parfums, les arômes, les colorants, les pigments, les agents matifiants, les agents rhéologiques, les conservateurs, les vitamines, les huiles essentielles, les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques et les filtres solaires.

8. Composition selon la revendication 7, **caractérisée en ce qu'**elle comprend comme additif un agent kératolytique, de préférence l'acide salicylique en association avec un actif favorisant la cicatrisation, de préférence l'huile essentielle d'arbre à thé.

9. Composition selon l'une quelconque des revendications précédentes, pour son utilisation dans une méthode de traitement de l'acné.

10. Méthode de traitement cosmétique de la peau comprenant au moins une application de la composition selon l'une des revendications 1 à 8 sur des imperfections cutanées toutes les 24 heures en vue d'améliorer l'apparence de la peau.

## Patentansprüche

1. Filmbildende topische Zusammensetzung, umfassend mindestens zwei Arten von Hydroxypropylcellulose, von denen mindestens eine ein Molekulargewicht von mindestens 800.000 und die andere ein Molekulargewicht von weniger als 400.000 aufweist, mindestens einen Weichmacher, mindestens ein organisches Lösungsmittel und mindestens ein anorganisches Lösungsmittel, **dadurch gekennzeichnet, dass**
(a) die Hydroxypropylcellulose mit einem Molekulargewicht von mindestens 800.000 von 0,2 bis 4 Gew.-% des Gesamtgewichts der Zusammensetzung ausmacht,
(b) die Hydroxypropylcellulose mit einem Molekulargewicht von weniger als 400.000 von 0,2 bis 20 Gew.-% des Gesamtgewichts der Zusammensetzung ausmacht,
(c) der Weichmacher 0,5 bis 10 Gew.-% des Gesamtgewichts der Zusammensetzung ausmacht,
(d) das anorganische Lösungsmittel 10 bis 60 Gew.-% des Gesamtgewichts der Zusammensetzung ausmacht,
(e) das organische Lösungsmittel 40 bis 80 Gew.-% des Gesamtgewichts der Zusammensetzung ausmacht.

2. Zusammensetzung nach Anspruch 1, **dadurch gekennzeichnet, daß** die Hydroxypropylcellulose mit einem Molekulargewicht von mindestens 800.000 in einer Menge von 1 bis 3 Gew.-% vorliegt, und die Hydroxypropylcellulose mit einem Molekulargewicht von weniger als 400.000 in einer Menge von 2 bis 6 Gew.-% vorliegt, bezogen auf das Gesamtgewicht der Zusammensetzung.

3. Zusammensetzung nach einem der Ansprüche 1 oder 2, **dadurch gekennzeichnet, dass** der Weichmacher ausgewählt ist aus Glykolen, insbesondere Glycerin.

4. Zusammensetzung nach einem der vorhergehenden Ansprüche, **dadurch gekennzeichnet, dass** das organische Lösungsmittel 50 bis 70 Gew.-% des Gesamtgewichts der Zusammensetzung ausmacht.

5. Zusammensetzung nach einem der vorhergehenden Ansprüche, **dadurch gekennzeichnet, dass** das anorganische Lösungsmittel 20 bis 40 Gew.-% des Gesamtgewichts der Zusammensetzung ausmacht.

6. Zusammensetzung nach einem der vorhergehenden Ansprüchen, **dadurch gekennzeichnet, dass** das organische Lösungsmittel ausgewählt ist aus Alkoholen, die bei Raumtemperatur flüssig sind, wie Ethanol, Isopropanol, Diacetonalkohol, 2-Butoxyethanol, Cyclohexanol und Mischungen davon, und vorzugsweise Ethanol.

7. Zusammensetzung nach einem der vorhergehenden Ansprüche, **dadurch gekennzeichnet, dass** sie pharmazeutisch annehmbare Zusatzstoffe umfasst, wie Duftstoffe, Geschmacksstoffe, Farbstoffe, Pigmente, Mattierungsmittel, Rheologiemittel, Konservierungsmittel, Vitamine, ätherische Öle, antibakterielle Mittel, Antiseptika, antivirale Mittel, antimykotische Mittel, Schmerzmittel, Entzündungshemmer, heilungsfördernde Mittel, Feuchtigkeitsmittel, Depigmentierungsmittel, keratolytische Mittel, umstrukturierende Wirkstoffe, Anästhetika und Sonnenschutzfilter.

8. Zusammensetzung nach Anspruch 7, **dadurch gekennzeichnet, dass** es als Zusatzstoff ein keratolytisches Mittel, vorzugsweise Salicylsäure, in Kombination mit einem heilungsfördemden Mittel, vorzugsweise dem ätherischen Öl des Teebaums, enthält.

9. Zusammensetzung nach einem der vorhergehenden Ansprüche zur Verwendung in einem Verfahren zur Behandlung von Akne.

10. Verfahren zur kosmetischen Behandlung der Haut, umfassend mindestens eine Anwendung der Zusammensetzung nach einem der Ansprüche 1 bis 8 auf kutane Unvollkommenheiten alle 24 Stunden, um das Erscheinungsbild der Haut zu verbessern.

## Claims

1. Film-forming topical composition comprising at least two grades of hydroxypropylcellulose of which at least one has a molecular weight of at least 800,000 and the other has a molecular weight less than 400,000, at least one plasticiser, at least one organic solvent and at least one inorganic solvent, **characterised in that**:
(a) the hydroxypropylcellulose having a molecular weight of at least 800,000 represents from 0.2 to 4% by weight of the total weight of the composition,
(b) the hydroxypropylcellulose having a molecular weight less than 400,000 represents from 0.2 to 20% by weight of the total weight of the composition,
(c) the plasticiser represents from 0.5 to 10% by weight of the total weight of the composition,
(d) the inorganic solvent represents 10 to 60% by weight of the total weight of the composition,
(e) the organic solvent represents 40 to 80% by weight of the total weight of the composition.

2. Composition according to claim 1, **characterised in that** the hydroxypropylcellulose having a molecular weight of at least 800,000 is present at a content ranging from 1 to 3% by weight, and the hydroxypropylcellulose having a molecular weight less than 400,000 by weight is present at a content ranging from 2 to 6% by weight with respect to the total weight of the composition.

3. Composition according to one of claims 1 or 2, **characterised in that** the plasticiser is chosen from among glycols, particularly glycerine.

4. Composition according to any one of the preceding claims, **characterised in that** the organic solvent represents from 50 to 70% by weight of the total weight of the composition.

5. Composition according to any one of the preceding claims, **characterised in that** the inorganic solvent represents from 20 to 40% by weight of the total weight of the composition.

6. Composition according to any one of the preceding claims, **characterised in that** the organic solvent is chosen among liquid alcohols at ambient temperature such as ethanol, isopropanol, diacetone alcohol, 2-butoxyethanol, cyclohexanol and mixtures thereof, and preferably ethanol.

7. Composition according to any one of the preceding claims, **characterised in that** it comprises pharmaceutically acceptable additives, such as perfumes, aromas, colorants, pigments, mattifying agents, rheological agents, preservatives, vitamins, essential oils, antibacterial agents, antiseptics, antivirals, antifungal agents, analgesics, anti-inflammatories, healing promoting agents, moisturising agents, depigmenting agents, keratolytic agents, restructuring agents, anaesthetics and sun filters.

8. Composition according to claim 7, **characterised in that** it comprises as an additive a keratolytic agent, preferably salicylic acid in association with a healing promoting agent, preferably tea tree essential oil.

9. Composition according to any one of the preceding claims, for use thereof in a method for treating acne.

10. Cosmetic skin treatment method comprising at least one application of the composition according to one of claims 1 to 8 on skin imperfections every 24 hours with a view to enhancing the appearance of the skin.
